# EUROPÄISCHE PATENTSCHRIFT

(11) **EP 3 329 950 B1**
(45) Veröffentlichungstag und Bekanntmachung des Hinweises auf die Patenterteilung: **04.09.2019**
(21) Anmeldenummer: 16201816.2
(22) Anmeldetag: 01.12.2016
(51) Int. Cl.: A61M 1/10

(54) **KANÜLE, KANÜLENSYSTEM UND BLUTPUMPENSYSTEM**
CANNULA, CANNULA SYSTEM AND BLOOD PUMP SYSTEM
CANULE, SYSTÈME CANULE ET SYSTÈME DE POMPE À SANG

(43) Veröffentlichungstag der Anmeldung: 06.06.2018
(73) Patentinhaber: Berlin Heart GmbH, 12247 Berlin (DE)
(72) Erfinder: PHILLIPS, Daniel, 10249 Berlin (DE); STOLLIN, Markus, 12169 Berlin (DE)
(74) Vertreter: Pfenning, Meinig & Partner mbB

(56) Entgegenhaltungen:
- EP-A1- 2 000 159
- EP-A1- 3 087 950
- WO-A1-2012/127145
- US-A- 3 409 913

## Beschreibung

Die Erfindung betrifft eine Kanüle zum Leiten einer Flüssigkeit, insbesondere Blut. Die Erfindung betrifft außerdem ein Kanülensystem, das eine solche Kanüle umfasst. Die Erfindung betrifft auch ein Blutpumpensystem, das ein solches Kanülensystem umfasst.

Kanülen werden in vielen Bereichen der Technik zum Leiten von Flüssigkeiten eingesetzt. Beispielsweise werden Kanülen in der Medizintechnik zum Leiten körpereigener Flüssigkeiten, wie etwa Blut, verwendet. Eine Kanüle kann beispielsweise als eine implantierbare Gefäßprothese ausgestaltet sein und als Ersatz für ein natürliches Blutgefäß dienen (siehe z.B. EP2000159).

In vielen Anwendungen, wie beispielsweise im Fall von Gefäßprothesen, ist es wichtig, dass auf möglichst schnelle und einfache Weise eine sichere Verbindung zwischen der Kanüle und einem anderen Hohlkörper hergestellt werden kann, wie beispielsweise mit einem Pumpenauslass einer Blutpumpe.

Es liegt der vorliegenden Erfindung somit die Aufgabe zugrunde, eine Kanüle vorzuschlagen, die auf möglichst einfache, schnelle und sicherer Weise mit einem anderen Hohlkörper verbunden werden kann. Ferner soll ein entsprechendes Kanülensystem mit einer solchen Kanüle sowie ein Blutpumpensystem mit einem solchen Kanülensystem vorgeschlagen werden.

Diese Aufgabe wird durch eine Kanüle gemäß dem Hauptanspruch sowie durch ein Kanülensystem und ein Blutpumpensystem gemäß den nebengeordneten Patentansprüchen gelöst. Weiterentwicklungen und besondere Ausführungsbeispiele dieser Gegenstände ergeben sich aus den abhängigen Ansprüchen sowie aus der nachfolgenden Beschreibung und den Figuren.

Die hier vorgeschlagene Kanüle zum Leiten einer Flüssigkeit, insbesondere einer körpereigenen Flüssigkeit wie beispielsweise Blut, umfasst ein Schlauchelement mit einem vorderen Endbereich und einem hinteren Endbereich. Das Schlauchelement definiert in seinem Innern einen Kanal zum Leiten der Flüssigkeit. Der Kanal verläuft axial durch das Schlauchelement hindurch von dem vorderen Endbereich des Schlauchelements bis zu dem hinteren Endbereich des Schlauchelements. Typischerweise verläuft der Kanal von einem vordersten Ende (des vorderen Endbereichs) des Schlauchelements bis zu einem hintersten Ende (des hinteren Endbereichs) des Schlauchelements.

Der vordere Endbereich des Schlauchelements definiert typischerweise einen Aufnahmebereich für einen Hohlkörper, mit dem die Kanüle verbunden werden soll. Dieser Hohlkörper, der ein Bestandteil des vorgeschlagenen Kanülensystems ist, ist derart ausgestaltet, dass er durch eine vordere Eingangsöffnung des Kanals in den Kanal eingeschoben werden kann, wie weiter unten beschrieben wird. Die vordere Eingangsöffnung des Kanals wird im Folgenden auch als vordere Öffnung des Kanals bezeichnet. Die vordere Öffnung des Kanals befindet sich typischerweise an dem oben genannten vordersten Ende des Schlauchelements.

Eine entsprechende Verbindung mit einem weiteren Hohlkörper kann auch für den hinteren Endbereich des Schlauchelements gelten. Alternativ kann aber auch vorgesehen sein, dass der hintere Endbereich des Schlauchelements mit einem Blutgefäß verbunden wird, beispielsweise durch Vernähen mit dem Blutgefäß oder auf eine andere Weise.

Hier und im Folgenden bedeutet "axial" entlang der jeweiligen Längsausdehnung eines Elements (beispielsweise des Schlauchelements oder des Hohlkörpers). Entsprechend bedeutet "radial" senkrecht zur Längsausdehnung des jeweiligen Elements. Entsprechend beziehen sich außerdem Begriffe wie "vorne", "vor", "vorderer", vorderster" bzw. "hinten", "hinter", "hinterer", "hinterster" etc. jeweils auf eine Anordnung in axialer Richtung, also entlang der jeweiligen Längsausdehnung eines Elements.

Typischerweise ist das Schlauchelement aus einem weichen und/oder flexiblen Material gebildet. Beispielsweise kann das Schlauchelement aus einem Graftmaterial gebildet sein und/oder kann beispielsweise eine textile schlauchförmige Trägerstruktur aufweisen, wie weiter unten näher beschrieben ist.

Die Kanüle umfasst außerdem einen Spannring, der mit dem vorderen Endbereich des Schlauchelements axial überlappt und mit dem vorderen Endbereich des Schlauchelements fest verbunden ist. Typischerweise ist der Spannring koaxial zu dem Schlauchelement angeordnet. Typischerweise umläuft der Spannring den Kanal im vorderen Endbereich des Schlauchelements konzentrisch. Typischerweise grenzt eine radial innere Oberfläche des Spannrings an eine radial äußere Oberfläche des Schlauchelements an. Es ist prinzipiell aber auch eine umgekehrte Konfiguration möglich, bei der eine radial äußere Oberfläche des Spannrings an eine radial innere Oberfläche des Schlauchelements angrenzt.

Allgemein werden hier zwei Elemente, wie beispielsweise also das Schlauchelement und der Spannring, als fest verbunden bezeichnet, wenn sie nicht zerstörungsfrei voneinander getrennt werden können. Derartige feste Verbindungen können beispielsweise stoffschlüssig (etwa durch Verkleben oder Verschmelzen) oder formschlüssig sein (etwa durch Vernähen). Im Gegensatz hierzu werden zwei Elemente als lösbar verbunden bezeichnet, wenn diese Elemente zerstörungsfrei voneinander getrennt werden können. So wird beispielsweise durch das Einschieben des genannten Hohlkörpers in den Kanal im vorderen Endbereich des Schlauchelements zwischen der Kanüle und diesem Hohlkörper eine lösbare Verbindung zwischen der Kanüle und dem Hohlkörper hergestellt, die beispielsweise durch Reibungskräfte und/oder Rastkräfte mechanisch stabilisiert sein kann, wie weiter unten näher beschrieben wird.

Ein Grundzustand der Kanüle ist dadurch definiert, dass auf die Kanüle als ganzes, oder zumindest auf den vorderen Endbereich des Schlauchelements und auf den Spannring, keine äußeren Kräfte einwirken. Als äußere Kräfte werden solche Kräfte bezeichnet, die auf die Kanüle von anderen Körpern, wie beispielsweise von dem oben genannten Hohlkörper, ausgeübt werden. Die Kraft, die also der gegebenenfalls in den vorderen Endbereich des Schlauchelements eingeschobene Hohlkörper auf die Kanüle ausübt, ist in diesem Sinne eine äußere Kraft. In dem Grundzustand der Kanüle ist in den vorderen Endbereich des Schlauchelements also insbesondere nicht der genannte Hohlkörper eingeschoben. Dieser kann nämlich beispielsweise auf den vorderen Endbereich des Schlauchelements und/oder auf den Spannring eine (äußere) Kraft ausüben, wie beispielsweise eine radial nach außen gerichtete Kraft.

In dem Grundzustand der Kanüle wirken somit ausschließlich innere Kräfte der Kanüle auf die Kanüle oder zumindest auf den vorderen Endbereich des Schlauchelements und auf den Spannring, ein. Als innere Kräfte der Kanüle werden also solche Kräfte verstanden, die verschiedene Teile der Kanüle wechselseitig aufeinander ausüben. In dem Grundzustand der Kanüle kompensieren sich die inneren Kräfte gegenseitig, so dass sich die Form der Kanüle zeitlich nicht (oder nur vernachlässigbar geringfügig) ändert.

In dem Grundzustand Kanüle ist der vordere Endbereich des Schlauchelements, vorzugsweise zumindest das vorderste Ende des Schlauchelements, durch eine von dem Spannring auf den vorderen Endbereich des Schlauchelements ausgeübte radial nach außen gerichtete (innere) Kraft vorgespannt. Vorzugsweise übt der Spannring die genannte radiale Kraft zumindest auf das vorderste Ende des Schlauchelements aus. In dem Grundzustand der Kanüle ist die Kanüle also insbesondere auch dann wie beschrieben vorgespannt, wenn der genannte Hohlkörper in den vorderen Endbereich des Schlauchelements nicht eingeschoben ist.

Durch diese Vorspannung ist die Form des Schlauchelements im vorderen Endbereich, vorzugsweise zumindest am vordersten Ende des Schlauchelements, stabilisiert. Hierdurch wird die Herstellung einer Verbindung mit einem Hohlkörper vereinfacht, insbesondere wenn das Schlauchelement in dem vorderen Endbereich aus einem weichen und/oder flexiblen Material gebildet ist.

Insbesondere kann der vordere Endbereich des Schlauchelements, vorzugsweise zumindest das vorderste Ende des Schlauchelements, durch den Spannring elastisch aufgeweitet sein. Durch die Aufweitung kann ein größerer Durchmesser der vorderen Eingangsöffnung des Kanals am vordersten Ende des Schlauchelements erzielt werden. Die vordere Öffnung kann beispielsweise durch das vorderste Ende des Schlauchelements selbst oder durch den Spannring (sofern dieser im Kanal angeordnet ist, wie oben beschrieben worden ist) radial umgrenzt sein. Durch die vergrößerte vordere Öffnung kann die Herstellung einer Verbindung mit dem genannten Hohlkörper vereinfacht werden. Beispielsweise kann die vordere Öffnung beim Einführen des Hohlkörpers in den Kanal leichter gefunden werden. Außerdem kann durch die Aufweitung das axiale Einführen des Hohlkörpers in den Kanal kontrollierter und mit einer kleineren axialen Kraft durchgeführt werden.

Beispielsweise ist es möglich, dass in dem Grundzustand der Kanüle ein Innendurchmesser des Schlauchelements innerhalb des vorderen Endbereichs zur vorderen Öffnung des Kanals hin zunimmt. Beispielsweise kann in dem Grundzustand der Kanüle der Innendurchmesser des Schlauchelements innerhalb des vorderen Endbereichs zum Spannring hin zunehmen. Bei dem genannten Innendurchmesser kann es sich beispielsweise jeweils um einen kleinsten Innendurchmesser oder einen gemittelten Innendurchmesser des Schlauchelements handeln.

Typischerweise ragt der Spannring axial nicht über den vorderen Endbereich des Schlauchelements hinaus. Insbesondere ragt der Spannring typischerweise axial nicht nach vorne über ein vorderstes Ende des Schlauchelements hinaus, sondern endet an oder noch hinter diesem. Typischerweise umläuft der Spannring den Kanal im vorderen Endbereich des Schlauchelements ringsum und/oder konzentrisch. In einem Ausführungsbeispiel grenzt eine radial innere Oberfläche des Spannrings an eine radial äußere Oberfläche des Schlauchelements an. In diesem Beispiel ist der Spannring also außerhalb des Kanals angeordnet. In einem anderen Ausführungsbeispiel grenzt eine radial äußere Oberfläche an eine radial innere Oberfläche des Schlauchelements an. In diesem Beispiel ist der Spannring also (zumindest teilweise) innerhalb des Kanals angeordnet.

Der Spannring ermöglicht aufgrund seiner festen Verbindung mit dem Schlauchelement und seiner Anordnung im vorderen Endbereich des Schlauchelements insbesondere auch eine Übertragung von äußeren Kräften, insbesondere von axialen Zugkräften oder axialen Schiebekräften, auf das Schlauchelement. Typischerweise werden derartige äußere Kräfte auf das Schlauchelement beim Herstellen der Verbindung mit dem Hohlkörper ausgeübt, insbesondere also beim Aufschieben des Schlauchelements auf den Hohlkörper bzw. beim Einschieben des Hohlköpers in das Schlauchelement. Somit kann der Spannring auch die Funktion eines Kraftübertragungselements, insbesondere eines Zugelements, haben. Hierzu kann der Spannring optional eine radiale Verbreiterung aufweisen, welche beispielsweise über eine radial äußere Oberfläche des Schlauchelements radial hinausragt, um über diese Verbreiterung die Übertragung der oben genannten Kräfte auf den Spannring und über den Spannring auf das Schlauchelement zu ermöglichen.

Die Kanüle umfasst einen Dichtring. Typischerweise ist der Dichtring koaxial zu dem Schlauchelement und außerdem koaxial zu dem Spannring angeordnet. Typischerweise umläuft der Dichtring den Kanal im vorderen Endbereich des Schlauchelements ringsum und/oder konzentrisch. Typischerweise grenzt eine radial innere Oberfläche des Dichtrings an eine radial äußere Oberfläche des Schlauchelements an.

Der Dichtring ist derart positioniert, dass er mit dem vorderen Endbereich des Schlauchelements axial überlappt und axial hinter dem Spannring angeordnet ist. Dabei ist der Dichtring von dem Spannring typischerweise axial beabstandet. Beispielsweise kann ein hinteres Ende des Dichtrings axial über ein hinteres Ende des vorderen Endbereichs des Schlauchelements nach hinten hinaus ragen. Es ist aber auch möglich, dass eine axiale Position des hinteren Endes des Dichtrings mit einer axialen Position des hinteren Endes des vorderen Endbereichs des Schlauchelements übereinstimmt. Sofern die Kanüle den genannten Dichtring aufweist, wirken in dem oben definierten Grundzustand der Kanüle auch keine äußeren Kräfte auf den Dichtring ein. In dem Grundzustand kann beispielsweise ein Innendurchmesser des Schlauchelements am Spannring größer sein als ein Innendurchmesser des Schlauchelements am Dichtring. Bei den genannten Innendurchmessern kann es sich beispielsweise jeweils um kleinste Innendurchmesser handeln. Typischerweise weist das Schlauchelement am Dichtring und am Spannring eine etwa gleiche Wanddicke auf.

In dem Grundzustand der Kanüle kann sich ein Innendurchmesser des Dichtrings nach vorne hin, also zum vordersten Ende des Schlauchelements hin, vergrößern. Anders ausgedrückt kann sich der Dichtring im Grundzustand der Kanüle zum hinteren Endbereich des Schlauchelements hin verjüngen. Hierdurch wird der Hohlkörper beim axialen Einschieben in den Kanal des Schlauchelements in radialer Richtung geführt und eine Zentrierung des Hohlkörpers verbessert. Beispielsweise kann die Innenfläche des Dichtrings eine konische Form aufweisen.

Der Dichtring kann relativ zum Schlauchelement beispielsweise verschiebbar und/oder verdrehbar sein. Hierdurch kann einerseits ein einfacherer Zusammenbau der Kanüle ermöglicht werden. In vielen Fällen kann hierdurch aber auch das Einführen des Hohlkörpers in den Kanal des Schlauchelements vereinfacht werden. Beispielsweise kann das Schlauchelement beim Einführen des Hohlkörpers (beispielsweise aufgrund von Reibungskräften zwischen dem Schlauchelement und dem Hohlkörper) sich relativ zum Dichtring axial nach hinten verschieben, wodurch beispielsweise Falten des Schlauchelements im vorderen Endbereich entfernt oder verringert werden können, so dass das Schlauchelement im vorderen Endbereich, insbesondere am Dichtring, weitgehend faltenfrei an dem Hohlkörper anliegt. Hierdurch wird eine bessere Abdichtung erreicht und das Risiko der Bildung von Thromben reduziert.

Der Spannring kann mit dem Schlauchelement beispielsweise stoffschlüssig verbunden sein, beispielsweise durch Verkleben oder Verschmelzen des Spannrings mit dem Schlauchelement. Alternativ oder zusätzlich kann der Spannring mit dem Schlauchelement vernäht sein. Der Spannring kann beispielsweise Löcher aufweisen, durch die ein Nähfaden, der den Spannring mit dem Schlauchelement verbindet, hindurch verläuft.

Der Spannring ist typischerweise steifer als des Schlauchelement und auch steifer als der Dichtring (sofern vorhanden) ausgestaltet. Beispielsweise kann der Spannring aus einem festeren Material gebildet sein als das Schlauchelement und auch aus einem festeren Material gebildet sein als der Dichtring (sofern vorhanden). Typischerweise bestehen der Spannring und der Dichtring (sofern vorhanden) jeweils aus einem festeren Material als das Schlauchelement. Der Spannring, der Dichtring wie auch der weitere Dichtring können beispielsweise aus biokompatiblen oder hämokompatiblen Materialen gebildet sein, beispielsweise aus entsprechenden Polymeren, insbesondere aus Silikon.

Typischerweise kann das Schlauchelement beispielsweise aus einem biokompatiblen oder hämokompatiblen Material gebildet sein, beispielsweise aus einem Graftmaterial. Beispielsweise kann das Schlauchelement eine textile Trägerstruktur aufweisen, die beispielsweise schlauchförmig ausgestaltet sein kann. Die textile Trägerstruktur kann beispielsweise aus einem Polyestergewebe gebildet sein.

Die Kanüle kann außerdem ein Bedienelement aufweisen, welches typischerweise einen hülsenförmigen Grundkörper aufweist. Der hülsenförmige Grundkörper kann beispielsweise eine äußere Grifffläche aufweisen. Ferner kann der hülsenförmige Grundkörper einen Innenbereich definieren, in dem der vordere Endbereich des Schlauchelements, der Spannring und, sofern vorhanden, vorzugsweise auch der Dichtring angeordnet sind. Typischerweise weist der hülsenförmige Grundkörper auf einer den Innenraum definierenden inneren Oberfläche einen Aufnahmebereich für den Spannring sowie gegebenenfalls außerdem einen Aufnahmebereich für den Dichtring auf. Derartige Aufnahmebereiche können beispielsweise durch Ringnuten oder durch radial nach innen vorspringende Ausbuchtungen des hülsenförmigen Grundkörpers definiert sein. Beispielsweise kann auf eine feste Verbindung zwischen dem Dichtring und dem hülsenförmigen Grundkörper verzichtet werden. Beispielsweise kann der Dichtring ausschließlich formschlüssig und/oder kraftschlüssig mit dem hülsenförmigen Grundkörper verbunden sein. Das Gleiche kann auch für die Verbindung des Spannrings und des Schlauchelements mit dem hülsenförmigen Grundkörper gelten.

Das Bedienelement dient typischerweise dazu, die Kanüle manuell zu handhaben. Vorzugsweise kann durch (rein) manuelle Bedienung des Bedienelements die beschriebene Verbindung der Kanüle mit dem Hohlkörper hergestellt werden. Hierzu können über das Bedienelement insbesondere äußere Kräfte, insbesondere axiale Zugkräfte oder axiale Schiebekräfte, auf das Schlauchelement übertragen werden. Diese Kraftübertragung auf das Schlauchelement kann beispielsweise über den Spannring erfolgen, wie oben beschrieben worden ist. Hierzu kann beispielsweise vorgesehen sein, dass der Spannring durch einen der oben genannten Aufnahmebereiche des hülsenförmigen Grundkörpers, insbesondere durch eine Ringnut oder eine radial nach innen ragende Ausbuchtung, in axialer Richtung relativ zum hülsenförmigen Grundkörper gestützt ist. Im Fall der radial nach innen ragende Ausbuchtung beispielsweise ist diese typischerweise axial hinter dem Spannring oder axial hinter der oben genannten optionalen radialen Verbreiterung des Spannrings angeordnet, so dass mittels dieser Ausbuchtung die genannten axialen Zugkräfte oder axialen Schiebekräfte nach vorne auf den Spannring übertragen werden können.

Außerdem kann das Bedienelement einen oder mehrere flexible Rastarme (Federarme) aufweisen, welche typischerweise von einem vorderen Ende des hülsenförmigen Grundkörpers aus freitragend axial über den vorderen Endbereich des Schlauchelements hinweg ragen. Jeder der Rastarme kann beispielsweise einen radial nach innen gerichteten Rastzahn aufweisen, der dazu ausgestaltet sein kann, eine Rastverbindung mit einem jeweils korrespondierenden Gegenrastelement einzugehen. Das jeweilige Gegenrastelement kann beispielsweise auf einer äußeren Oberfläche des Hohlkörpers, der für eine Verbindung mit der Kanüle vorgesehen ist, angeordnet sein, wie weiter unten näher beschrieben wird.

Das hier vorgeschlagene Kanülensystem, umfasst eine Kanüle hier vorgeschlagener Art sowie den genannten Hohlkörper, der für eine Verbindung mit der Kanüle vorgesehen ist. Der Hohlkörper kann beispielsweise als ein Rohr, insbesondere als ein Pumpeneinlass oder als ein Pumpenauslass einer Blutpumpe, ausgestaltet sein. Der Hohlkörper definiert ebenfalls einen Kanal zum Leiten einer Flüssigkeit, wie etwa Blut. Der Hohlkörper ist typischerweise aus einem biokompatiblen oder hämokompatiblen Material gebildet, beispielsweise aus einem Metall, wie etwa einem Edelstahl oder einer Titanlegierung, oder aber aus einem Polymer, wie etwa einem Silikon.

Der Hohlkörper weist einen vorderen Endbereich auf. Der vordere Endbereich des Hohlkörpers ist derart ausgeformt, dass er durch die vordere Öffnung des Kanals des Schlauchelements hindurch in den Kanal des Schlauchelements eingeschoben werden kann, so dass der vordere Endbereich des Hohlkörpers mit dem vorderen Endbereich des Schlauchelements axial überlappt. Typischerweise haben der vordere Endbereich des Schlauchelements und der vordere Endbereich des Hohlkörpers eine gleichgroße axiale Ausdehnung. In dem eingeschobenen Zustand bilden die Kanäle des Schlauchelements und des Hohlkörpers einen durchgängigen Kanal.

Der Hohlkörper kann einen Anschlag aufweisen, bis zu dem der Hohlkörper in den Kanal einschiebbar ist. Der Anschlag selbst gehört somit nicht mehr zum vorderen Endbereich des Hohlkörpers, sondern ist axial hinter dem vorderen Endbereich des Hohlkörpers angeordnet. Der Anschlag definiert eine maximale axiale Tiefe, mit der der Hohlkörper in den Kanal eingeschoben werden kann. Der Anschlag kann beispielsweise in Form einer radialen Verbreiterung des Hohlkörpers ausgestaltet sein, beispielsweise in Form eines Stegs auf einer äußeren Oberfläche des Hohlkörpers.

Wie bereits oben erwähnt, kann der Hohlkörper mindestens ein Rastelement oder ein Gegenrastelement aufweisen zum Herstellen einer Rastverbindung zwischen der Kanüle und dem Rohr. Das mindestens eine Rastelement oder Gegenrastelement kann beispielsweise durch einen Oberflächenbereich des oben genannten Anschlags gebildet sein.

Vorzugsweise ist in dem Grundzustand der Kanüle ein Durchmesser, beispielsweise ein kleinster Durchmesser, der vorderen Öffnung des Kanals größer als ein Außendurchmesser des Hohlkörpers an einem vordersten Ende des vorderen Endbereichs des Hohlkörpers, Zusätzlich oder alternativ kann in dem Grundzustand der Kanüle der Durchmesser der vorderen Öffnung des Kanals des Schlauchelements, beispielsweise der kleinste Durchmesser, größer sein als ein größter Außendurchmesser des vorderen Endbereichs des Hohlkörpers, wobei der vordere Endbereich beispielsweise, wie oben beschrieben, durch den oben genannten Anschlag definiert sein kann.

Beispielsweise kann, wenn der vordere Endbereich, beispielsweise soweit es der genannte Anschlag erlaubt, in den Kanal eingeschoben ist, zwischen dem Schlauchelement und dem Hohlkörper ein freier Zwischenspalt bestehen, der den Hohlkörper beispielsweise ringförmig umläuft (Ringspalt). Der Zwischenspalt ist typischerweise innerhalb des Kanals des Schlauchelements und in einem axialen Überlappungsbereich des Spannelements und des Schlauchelements angeordnet.

Sofern der oben beschriebene Dichtring vorgesehen ist, ist dieser vorzugsweise derart angeordnet, dass er mit dem in den Kanal (ggf. bis zum Anschlag) eingeschobenen Endbereich des Hohlkörpers axial überlappt. In einem axialen Überlappungsbereich, in dem der vordere Endbereich des (ggf. bis zum Anschlag) eingeschobenen Hohlkörpers und der Dichtring sich axial überlappen, besteht zwischen dem Schlauchelement und dem Hohlkörper eine durch den Dichtring hervorgerufene gegenseitige (radiale) Anpresskraft, wodurch zwischen dem Hohlkörper und dem Schlauchelement eine Abdichtung bewirkt wird. Die Stärke dieser gegenseitigen Anpresskraft kann beispielsweise durch eine geeignete Wahl des Innendurchmessers des Dichtrings und/oder der Festigkeit des Dichtrings angepasst werden. Der Dichtring wird durch den eingeführten Hohlkörper typischerweise elastisch aufgedehnt. Sofern kein solcher Dichtring vorgesehen ist, kann eine gegenseitige Anpresskraft zwischen dem Schlauchelement und dem eingeführten Hohlkörper beispielsweise auch durch die Elastizität des (durch den Hohlkörper radial gedehnten) Schlauchelements bewirkt werden. Typischerweise erfolgt im axialen Überlappungsbereich mit dem Spannring keine radiale Dehnung des Schlauchelements durch den eingeführten Hohlkörper (sondern nur durch den Spannring). Eine radiale Dehnung des Schlauchelements durch den eingeführten Hohlkörper erfolgt, sofern überhaupt, dann typischerweise erst axial hinter dem Spannring.

Der vordere Endbereich des in den Kanal (bis zum Anschlag) eingeschobenen Hohlkörpers kann beispielsweise innerhalb oder an einem hinteren Ende des oben genannten axialen Überlappungsbereichs des Schlauchelements und des Dichtrings enden. Auf diese Weise kann verhindert werden, dass Flüssigkeit zwischen das Schlauchelement und den Hohlkörper gelangt (Taschenbildung).

Das Blutpumpensystem hier vorgeschlagener Art umfasst das hier vorgeschlagene Kanülensystem und eine Blutpumpe, typischerweise eine implantierbare Blutpumpe. Die Kanüle ist dann typischerweise als eine implantierbare Gefäßprothese ausgestaltet. Beispielsweise ist das Schlauchelement in diesem Fall aus einem Graftmaterial gebildet und weist beispielsweise eine textile Trägerstruktur auf, wie bereits beschrieben.

Die Blutpumpe weist typischerweise ein Pumpengehäuse auf. Das Pumpengehäuse weist einen Pumpeneinlass und einem Pumpenauslass auf, welche typischerweise rohrförmig ausgestaltet sind. Beispielsweise bildet der Hohlkörper des Kanülensystems den Pumpenauslass oder den Pumpenauslass der Blutpumpe. Weitere optionale Merkmale der Blutpumpe werden weiter unten im Zusammenhang konkreter Ausführungsbeispiele beschrieben.

Nachfolgend wird die hier vorgeschlagene Kanüle, das vorgeschlagene Kanülensystem und das vorgeschlagene Blutpumpensystem anhand von in Figuren 1 bis 10 schematisch dargestellten Ausführungsbeispielen näher beschrieben. Es zeigen:
- Figur 1: eine perspektivische Darstellung eines Blutpumpensystems hier vorgeschlagener Art,
- Figur 2: das in Figur 1 gezeigte Blutpumpensystem in einer Ansicht von oben,
- Figur 3: das in Figur 1 gezeigte Blutpumpensystem in einer Schnittdarstellung,
- Figur 4A: ein Kanülensystem des in Figur 1 gezeigten Blutpumpensystems in einer Längsschnittdarstellung,
- Figur 4B: eine Variante des in Figur 4A gezeigten Kanülensystems in einer Längsschnittdarstellung in einem verbundenen Zustand,
- Fign. 5A, 5B: eine seitliche Ansicht und eine perspektivische Ansicht auf einen Längsschnitt durch das in Figur 4A gezeigte Kanülensystem in einem getrennten Zustand,
- Figur 6A: ein Bedienelement einer Kanüle des in Figur 4A gezeigten Kanülensystems in einer Schnittdarstellung und in einer perspektivischen Darstellung,
- Figur 6B: ein Bedienelement einer Kanüle des in Figur 4B gezeigten Kanülensystems in einer Schnittdarstellung und in einer perspektivischen Darstellung,
- Figur 7A: einen Hohlkörper des in Figur 4A gezeigten Kanülensystems in einer kollabierten axialen Ansicht und in einer Längsschnittdarstellung,
- Figur 7B: einen Hohlkörper des in Figur 4B gezeigten Kanülensystems in einer kollabierten axialen Ansicht und in einer Längsschnittdarstellung,
- Figur 8: einen Dichtring des in Figur 4A oder in Figur 4B gezeigten Kanülensystems in einer Längsschnittdarstellung und in einer perspektivischen Darstellung,
- Figur 9: einen Spannring des in Figur 4A oder in Figur 4B gezeigten Kanülensystems in einer Längsschnittdarstellung und in einer perspektivischen Darstellung, und
- Figur 10: einen weiteren Dichtring des in Figur 4A oder in Figur 4B gezeigten Kanülensystems in einer Längsschnittdarstellung und in einer perspektivischen Darstellung.

Identische oder einander entsprechende Merkmale sind in den Figuren und in der nachfolgenden Beschreibung mit den gleichen Bezugszeichen versehen.

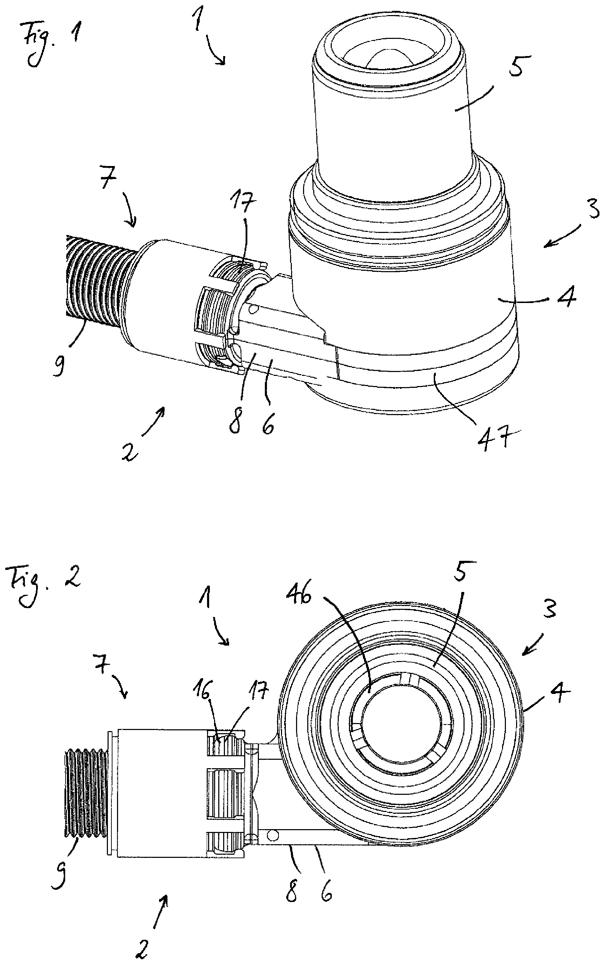

Figuren 1 bis 3 zeigen ein Blutpumpensystem 1 hier vorgeschlagener Art, das ein Kanülensystem 2 hier vorgeschlagener Art und eine Blutpumpe 3 umfasst. Die Blutpumpe 2 ist implantierbar und weist ein Pumpengehäuse 4 mit einem rohrförmigen Pumpeneinlass 5 und einem rohrförmigen Pumpenauslass 6 auf.

Das Kanülensystem 2 umfasst eine Kanüle 7, die als eine implantierbare Gefäßprothese ausgestaltet ist, sowie einen Hohlkörper 8, der in diesem Beispiel durch den rohrförmigen Pumpenauslass 5 gegeben ist.

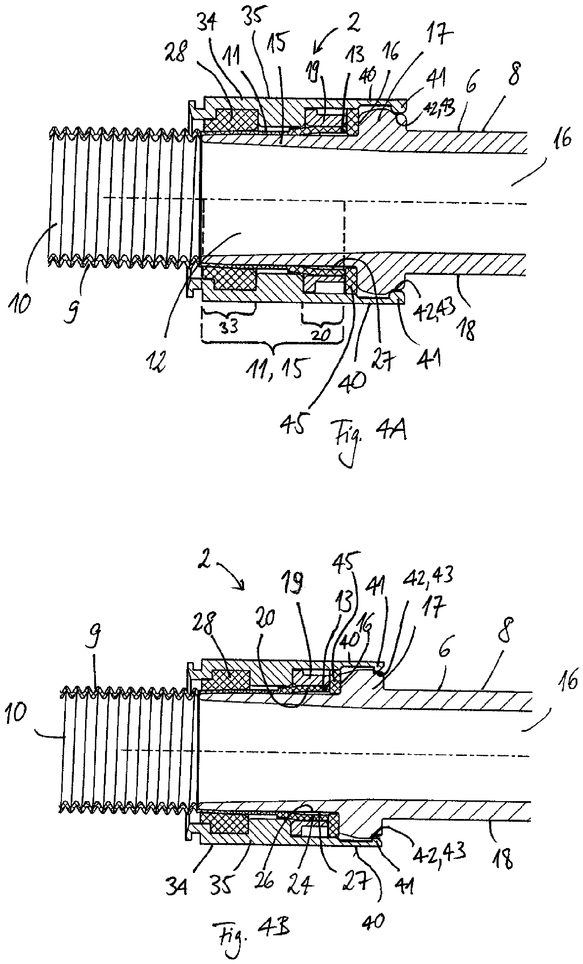

Wie in Figuren 1 bis 3 sowie auch in Figuren 4A und 4B gezeigt ist, ist die Kanüle 7 ist mit dem Hohlkörper 8 mittels einer Rastverbindung lösbar verbindbar, wie weiter unten detaillierter beschrieben wird.

Die Kanüle 7 umfasst ein Schlauchelement 9, das aus einem weichen und elastischen Material gebildet ist, vorliegend beispielsweise aus einem Graftmaterial mit einem textilen schlauchförmigen Träger aus einem Polyestergewebe (nicht dargestellt). Wie beispielsweise in Figuren 4A und 4B gezeigt ist, definiert das Schlauchelement 9 in seinem Innern einen Kanal 10 zum Leiten von Blut.

Wie in Figuren 4A und 4B außerdem gezeigt ist, weist das Schlauchelement 9 einen vorderen Endbereich 11 auf, der einen Aufnahmebereich 12 für den Hohlkörper 6 bzw. den Pumpenauslass 6 definiert und an dessen vordersten Ende 13 sich eine vordere Einlassöffnung 14 bzw. eine vordere Öffnung 14 des Kanals 10 befindet. Der Kanal 10 verläuft von dem vorderen Endbereich 11 bis zu einem hinteren Endbereich des Schlauchelements 9. In den Figuren 1 bis 5B ist das Schlauchelement 9 jeweils nur teilweise dargestellt, so dass der hintere Endbereich nicht abgebildet ist. Der hintere Endbereich des Schlauchelements 9 ist beispielsweise mit einem Blutgefäß verbindbar, beispielsweise durch Vernähen mit dem Blutgefäß, ggf. unter Verwendung eines geeigneten Nahtrings (ebenfalls nicht dargestellt).

Wie beispielsweise in Figuren 4A, 4B, 5A, 5B, 6A und 6B dargestellt ist, weist der Hohlkörper 8 ebenfalls einen vorderen Endbereich 15 auf, der derart ausgeformt und dimensioniert ist, dass er durch die vordere Öffnung 14 des Kanals 10 in den Kanal 10 des Schlauchelements 9 eingeschoben werden kann, so dass der vordere Endbereich 15 des Hohlkörpers 8 mit dem vorderen Endbereich 11 des Schlauchelements 9 vollständig axial überlappt. Im gezeigten Beispiel haben der vordere Endbereich 11 des Schlauchelements 9 und der vordere Endbereich 16 des Hohlkörpers 8 eine gleich große axiale Ausdehnung. Wie in Figur 4A gezeigt ist, definiert der Hohlkörper 8 ebenfalls einen Kanal 16 zum Leiten von Blut. In dem in Figuren 1 bis 3, 4A und 4B gezeigten eingeschobenen Zustand bilden die Kanäle 10, 16 des Schlauchelements 9 und des Hohlkörpers 8 einen durchgängigen Kanal.

Wie in Figuren 1 bis 5B und 7A und 7B gezeigt ist, weist der Hohlkörper 8 bzw. der Pumpenauslass 6 einen Anschlag 16 auf, bis zu dem der Hohlkörper 8 bzw. der Pumpenauslass 6 in den Kanal 10 eingeschoben werden kann. Der Anschlag 16 definiert somit eine maximale axiale Tiefe, mit der der Hohlkörper 8 bzw. der Pumpenauslass 6 in den Kanal 10 eingeschoben werden kann. Der Anschlag 18 ist beispielsweise in Form einer stegförmigen Verbreiterung 17 auf einer äußeren Oberfläche 18 des Hohlkörpers 8 bzw. Pumpenauslass 6 ausgestaltet.

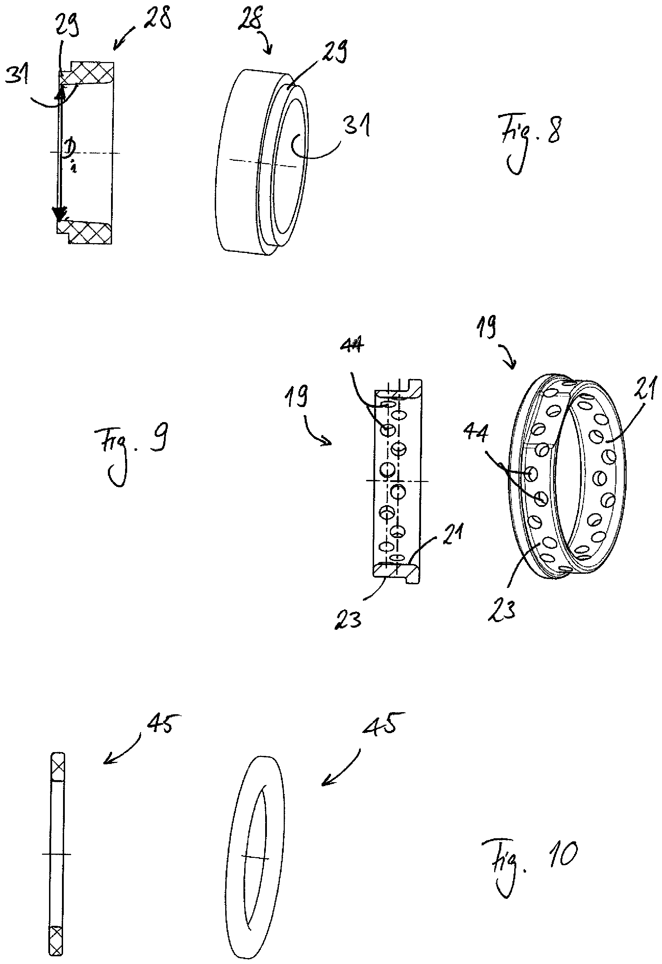

Die Kanüle 7 umfasst außerdem einen Spannring 19, der mit dem vorderen Endbereich 11 des Schlauchelements 9 innerhalb eines axialen Überlappungsbereichs 20 axial überlappt und mit dem vorderen Endbereich 11 des Schlauchelements 9 fest verbunden ist. Der Spannring 19 ist in Figur 9 noch einmal isoliert dargestellt.

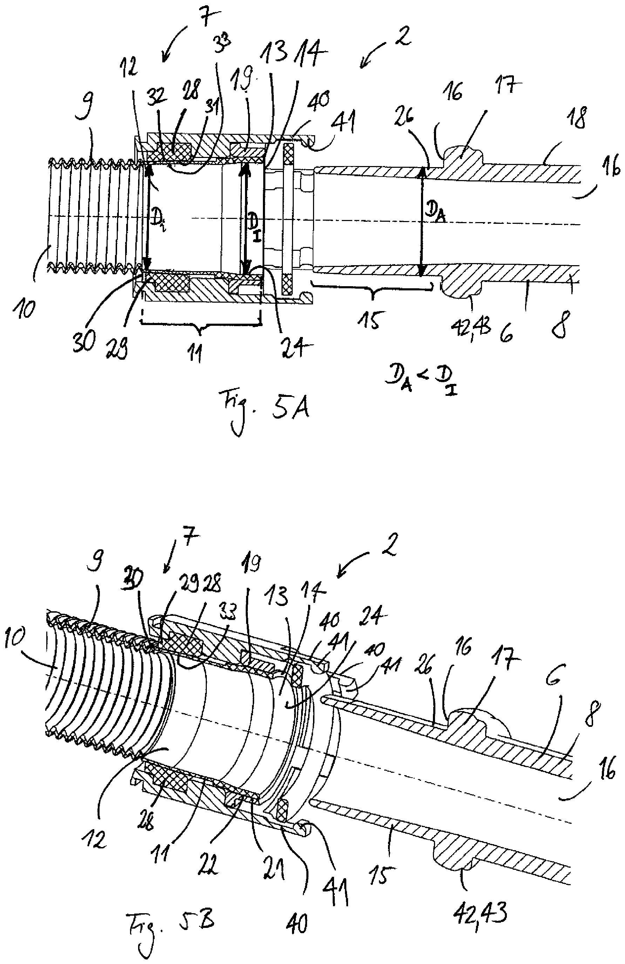

In einem Grundzustand der Kanüle 7, in dem auf den vorderen Endbereich 11 des Schlauchelements 9 und auf den Spannring 19 keine äußeren Kräfte, sondern lediglich zwischen diesen Elementen wechselseitig ausgeübte innere Kräfte wirken, ist das Schlauchelement 9 durch eine von dem Spannring 19 im axialen Überlappungsbereich 20 auf den vorderen Endbereich 11 des Schlauchelements 9, insbesondere auf das vorderste Ende 13 des Schlauchelements 9, ausgeübte radial nach außen gerichtete (innere) Kraft vorgespannt. In dem Grundzustand der Kanüle 7, also insbesondere auch dann, wenn der Hohlkörper 8 nicht in den vorderen Endbereich 11 des Schlauchelements eingeschoben ist, wie beispielsweise in Figuren 5A und 5B gezeigt ist, ist der vordere Endbereich 11 somit zumindest am vordersten Ende 13 des Schlauchelements 8 durch den Spannring 19 vorgespannt. Durch diese Vorspannung ist zum einen die Form des vordersten Endes 13 stabilisiert. Außerdem ist das vorderste Ende 13 des Schlauchelements 8 durch diese Vorspannung elastisch aufgeweitet, so dass ein Durchmesser der vorderen Öffnung 14 des Kanals 10, welche im gezeigten Beispiel durch das vorderste Ende 13 des Schlauchelements 9 radial umgrenzt wird, vergrößert ist.

Wie beispielsweise aus Figuren 4A und 5A ersichtlich ist, ragt der Spannring 19 axial nicht über den vorderen Endbereich 11 des Schlauchelements 9 hinaus, insbesondere also nicht axial nach vorne über das vorderste Ende 13 des Schlauchelements 13 hinaus, sondern endet an diesem. Der Spannring 19 umläuft sowohl den Kanal 10 im axialen Überlappungsbereich 20 als auch das Schlauchelement 9 ringsum und konzentrisch. Wie beispielsweise aus Figur 5B ersichtlich ist, grenzt eine radial innere Oberfläche 21 des Spannrings 19 an eine radial äußere Oberfläche 22 des Schlauchelements 9 an. In diesem Beispiel ist der Spannring 19 also außerhalb des Kanals 10 angeordnet. Prinzipiell wäre es aber auch möglich, dass eine radial äußere Oberfläche 23 des Spannrings 19 an eine radial innere Oberfläche 24 des Schlauchelements 9 angrenzt.

In diesem Beispiel wäre der Spannring 19 also (zumindest teilweise) innerhalb des Kanals 10 angeordnet.

In dem Grundzustand nimmt ein Innendurchmesser des Schlauchelements 9, insbesondere ein kleinster Innendurchmesser des Schlauchelements 9, innerhalb des vorderen Endbereichs 11 zur vorderen Öffnung 14 des Kanals 10 hin zu. Beispielsweise nimmt in dem Grundzustand der Kanüle 7 der kleinste Innendurchmesser des Schlauchelements innerhalb des vorderen Endbereichs 11 zum Spannring 19 hin zu (siehe beispielsweise Figuren 4A und 5A).
In dem gezeigten Beispiel ist in dem Grundzustand der Kanüle 7 ein Durchmesser der vorderen Öffnung 14 des Kanals 7, also ein (kleinster) Innendurchmesser D_{I} des vordersten Endes 13 des Schlauchelements 9, größer als ein größter Außendurchmesser D_{A} des Hohlkörpers 8 in dem vorderen Endbereich 15 des Hohlkörpers 8, D_{A} < D_{I}. Auf diese Weise wird ein Einführen eines vordersten Endes 25 des Hohlelements 8 in die vordere Öffnung 14 des Kanals 10 des Schlauchelements 9 vereinfacht. Wenn der vordere Endbereich 15 des Hohlkörpers 8 vollständig, also so weit wie es der Anschlag 16 erlaubt, in den Kanal 10 eingeschoben ist, besteht in dem axialen Überlappungsbereich 20 zwischen der inneren Oberfläche 24 des Schlauchelements 9 und einer äußeren Oberfläche 26 des vorderen Endbereichs 25 des Hohlkörpers 8 ein freier Zwischenraum 27 in Form eines Ringspalts, der den Hohlkörper 8 innerhalb des Kanals 10 ringförmig umläuft (siehe beispielsweise Figuren 4A und 4B).

Wie in Figuren 4A, 4B, 5A und 5B gezeigt ist, umfasst die Kanüle 7 außerdem einen mit dem vorderen Endbereich 11 des Schlauchelements 9 axial überlappenden Dichtring 28. Der Dichtring 28 ist axial hinter dem Spannring 19 angeordnet und von diesem axial beabstandet. In dem gezeigten Beispiel stimmt eine axiale Position eines hinteren Endes 29 des Dichtrings 28 mit einer axialen Position eines hinteren Endes 30 des vorderen Endbereichs 11 des Schlauchelements 9 überein. Zudem umläuft der Dichtring 28 den Kanal 10 ringsum und konzentrisch. Zudem grenzt eine radial innere Oberfläche 31 des Dichtrings 28 an eine radial äußere Oberfläche 32 des Schlauchelements 9 an. Der Dichtring 28 ist relativ zum Schlauchelement 9 verschiebbar und verdrehbar.

In dem Grundzustand der Kanüle 7, in dem auch keine äußeren Kräfte auf den Dichtring 28 einwirken, ist der Innendurchmesser D_{I} des Schlauchelements 9 am Spannring 19 größer als ein Innendurchmesser Dᵢ des Schlauchelements 9 am Dichtring 28. Bei den genannten Innendurchmessern D_{I}, Dᵢ handelt es sich jeweils um kleinste Innendurchmesser in dem jeweils betrachteten axialen Bereich.

Wie in den in Figur 8 gezeigten Darstellungen des Dichtrings 28 deutlich zu erkennen ist, weitet sich der Dichtring 28 im Grundzustand nach vorne hin, also zum vordersten Ende 13 des Schlauchelements 8 hin, auf und verjüngt sich entsprechend in der entgegengesetzten Richtung. Die Innenfläche 31 des Dichtrings 28 (also dessen radial innere Oberfläche) weist beispielsweise eine konische Form auf.

Wie in Figuren 4A und 4B zu erkennen ist, ist der Dichtring 28 derart angeordnet, dass er mit dem in den Kanal 10 bis zum Anschlag 16 eingeschobenen Endbereich 15 des Hohlkörpers 8 in einem axialen Überlappungsbereich 33 axial überlappt. In diesem axialen Überlappungsbereich 33 besteht zwischen dem Schlauchelement 9 und dem eingeführten Hohlkörper 9 eine durch den Dichtring 28 hervorgerufene gegenseitige (radiale) Anpresskraft, wodurch zwischen dem Hohlkörper 8 und dem Schlauchelement 9 eine (radiale) Abdichtung bewirkt wird. Die Stärke dieser gegenseitigen Anpresskraft ist durch eine geeignete Wahl des Innendurchmessers Dᵢ des Dichtrings, des Außendurchmesser D_{A} des vorderen Endbereichs 15 des Hohlkörpers 8 in dem axialen Überlappungsbereich 33 und der Festigkeit des Dichtrings 28 angepasst.

Der vordere Endbereich 15 des in den Kanal 9 bis zum Anschlag 16 eingeschobenen Hohlkörpers 8 ragt nicht über das hintere Ende 29 des Dichtrings 28 hinaus, sondern endet an dem hinteren Ende 29 des Dichtrings 28, also am hinteren Ende des oben genannten axialen Überlappungsbereichs 33. Auf diese Weise wird verhindert, dass Blut zwischen das Schlauchelement 9 und den Hohlkörper 8 gelangt (Taschenbildung des Schlauchelements), wodurch das Risiko der Entstehung von Thromben reduziert wird.

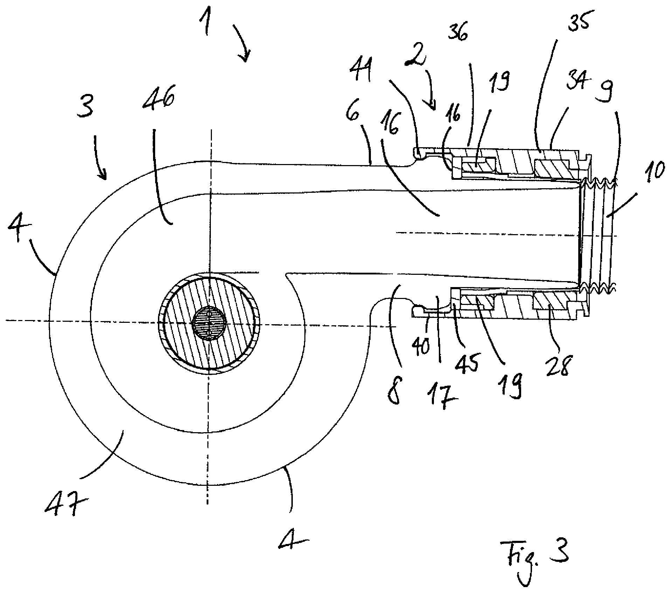

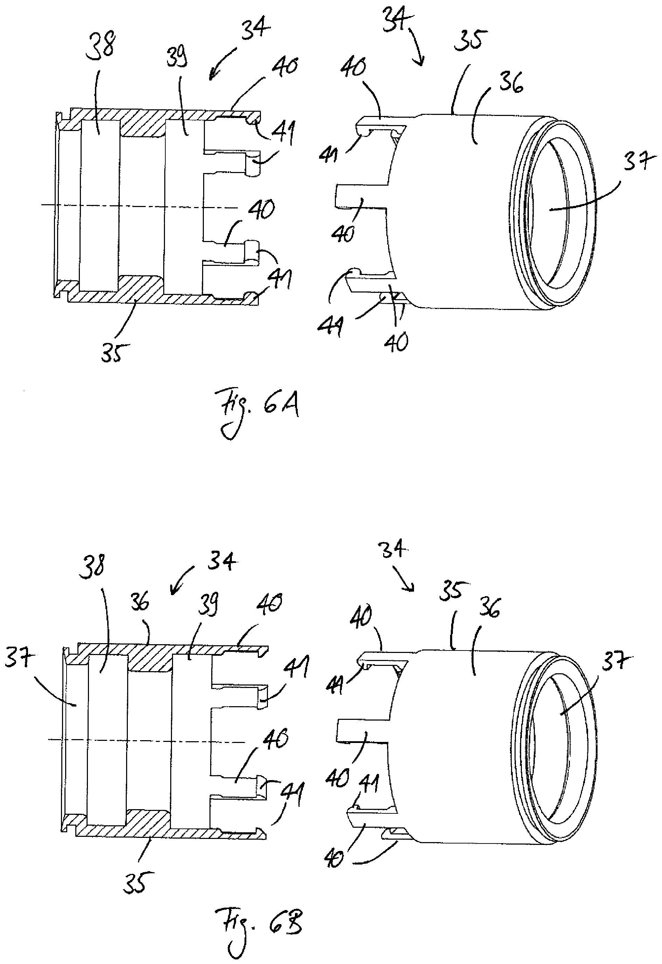

Die Kanüle 7 umfasst außerdem ein Bedienelement 34 mit einem hülsenförmigen Grundkörper 35. Der hülsenförmige Grundkörper 35 weist eine äußere Grifffläche 36 auf und definiert einen Innenbereich 37 (siehe beispielsweise Figuren 6A und 6B, in denen das Bedienelement 34 isoliert dargestellt ist). Wie in Figuren 3, 4A, 4B, 5A und 5B gezeigt ist, sind in dem Innenbereich 37 der vordere Endbereich 11 des Schlauchelements 9, der Spannring 19 und der Dichtring 28 aufgenommen. Hierzu weist der hülsenförmige Grundkörper 35 auf einer den Innenraum definierenden inneren Oberfläche Aufnahmebereiche 38, 39 für den Spannring 19 und für den Dichtring 28 auf. Diese Aufnahmebereiche 38, 39 sind beispielsweise durch Ringnuten bzw. oder durch radial nach innen vorspringende Ausbuchtungen des hülsenförmigen Grundkörpers 35 definiert.

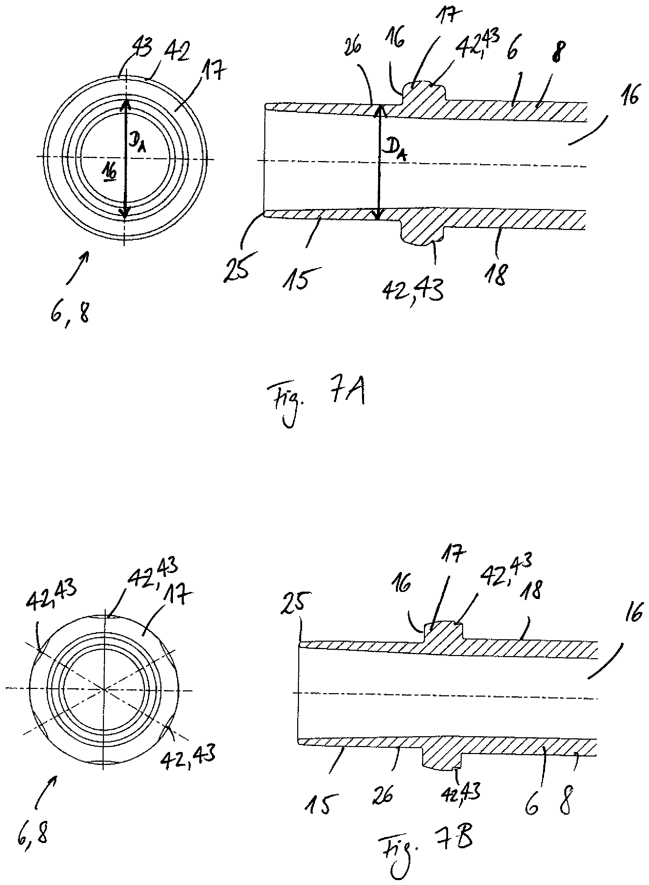

Außerdem weist das Bedienelement 34 mehrere als Rastarme ausgestaltete Rastelemente 40 auf, welche von einem vorderen Ende des hülsenförmigen Grundkörpers 35 aus freitragend axial über den vorderen Endbereich 11 des Schlauchelements 9 hinweg ragen (siehe beispielsweise Figur 3). Jedes der Rastelemente 40 weist einen radial nach innen gerichteten Rastzahn 41 auf, der dazu ausgestaltet ist, eine Rastverbindung mit einem korrespondierenden Gegenrastelement 42 des Hohlkörpers 8 einzugehen, sobald dieser bis zum Anschlag 16 in den Kanal 10 eingeschoben ist. Diese Gegenrastelemente 42 sind in diesem Beispiel als Rastflächen 43 auf der Oberfläche 18 des Hohlkörpers 8 ausgestaltet, vorliegend beispielsweise auf der Verbreiterung 17, welche den Anschlag 16 bildet (siehe beispielsweise Figuren 7A und 7B).

In dem in Figur 7A gezeigten Beispiel sind diese Rastflächen 43 abgerundet, ebenso wie die in Figur 6A gezeigten hierzu korrespondierenden Rastzähne 42. Eine solche abgerundete Ausformung dieser Rastpartner erlaubt, ebenso wie eine ebenfalls mögliche Fasenschräge, ein Lösen der Rastverbindung durch axiales Auseinanderziehen der Kanüle 7 und des Hohlkörpers 8.

Dahingegen sind in der in Figur 7B gezeigten Variante des Hohlkörpers 8 diese Rastflächen 43 ebenso wie die in Figur 6B gezeigten hierzu korrespondierenden Rastzähne 42 in axialer Richtung scharfkantig ausgeformt. Ein Lösen der Rastverbindung erfordert in dieser Variante somit zusätzlich zu dem relativen axialen Auseinanderziehen auch eine relative Drehbewegung der Kanüle 7 zum Hohlkörper 8. Durch diese relative Drehbewegung, im vorliegenden Beispiel um etwa 30° ausgehend von einer stabilen Raststellung, gleiten die Rastzähne 41 über die Rastflächen 43, wodurch die Rastelemente 40 radial soweit auseinandergespreizt werden, dass sie über die Verbreiterung 17 axial abgezogen werden können.

In den in Figur 9 gezeigten Darstellungen des Spannrings 19 ist zu erkennen, dass der Spannring mehrere Durchgangslöcher 44 aufweist. Durch diese verläuft im hier beschriebenen Beispiel der Kanüle 7 ein Nähfaden (nicht dargestellt), mit dem der Spannring 19 mit dem Schlauchelement 9 vernäht ist. Zusätzlich oder alternativ wäre es auch möglich, den Spannring 19 mit dem Schlauchelement durch Verkleben oder Verschmelzen fest zu verbinden.

Der Spannring 19 ist in diesem Beispiel steifer als das Schlauchelement 9 und auch steifer als der Dichtring 28 ausgestaltet. Hierzu ist der Spannring 19 beispielsweise aus einem festeren Material gebildet als das Schlauchelement 9 und als der Dichtring 28.

Die Kanüle 7 umfasst in dem gezeigten Ausführungsbeispiel außerdem einen optionalen weiteren Dichtring 45, der in den in Figur 10 gezeigten Darstellungen zur Verdeutlichung noch einmal isoliert abgebildet ist. Dieser weitere Dichtring 45 ist vorzugsweise axial vor dem Spannring 19 sowie koaxial zum Spannring 19 und dem ersten Dichtring 28 angeordnet. Zudem ist der weitere Dichtring 45 beispielsweise innerhalb des Innenbereichs 37 des Hohlkörpers 35 angeordnet. Im verbundenen Zustand, wenn also, wie in Figuren 4A und 4B gezeigt ist, der Hohlkörper 8 bis zum Anschlag 16 in den Kanal 10 eingeführt ist, ist der weitere Dichtring 45 axial zwischen dem Anschlag 16 und dem Spannring 19 angeordnet und umläuft den Kanal 16 des Hohlkörpers 8 konzentrisch.

Der Spannring 19, der Dichtring 28 und der weitere Dichtring 45 sind jeweils aus einem hämokompatiblen Material gebildet, beispielsweise jeweils aus einem Polymer, wie etwa einem Silikon. Das Bedienelement 34 ist ebenfalls aus einem hämokompatiblen Material gebildet, wie beispielsweise aus einem Edelstahl oder einer Titanlegierung.

Sobald die oben beschriebene Rastverbindung zwischen der Kanüle 7 und dem Hohlkörper 6 hergestellt ist, pressen der Anschlag 16 und der Spannring 19 jeweils axial gegen den Dichtring 45, so dass durch den Dichtring 45 eine zusätzliche Abdichtung der Verbindung bewirkt wird. Der weitere Dichtring 45 ist beispielsweise aus dem gleichen Material gebildet wie der erstgenannte Dichtring 28.

Prinzipiell kann die hier vorgeschlagene Kanüle 7 aber auch ohne den weiteren Dichtring 45 ausgestaltet sein. Dann kann beispielsweise vorgesehen sein, dass der Hohlkörper 8 soweit in den Kanal eingeführt wird, bis der Spannring 19 den Anschlag 16 kontaktiert und gegebenenfalls axial gegen diesen presst, wenn die Rastverbindung hergestellt ist.

Prinzipiell kann die vorgeschlagene Kanüle 7 außerdem einen Knickschutz umfassen, der beispielsweise an einem hinteren Ende des Bedienelements 34 bzw. dessen hülsenförmigen Grundkörper 35 befestigt sein kann.

Neben dem Vorteil, dass das gezeigte Kanülensystem 2 eine einfache und sichere manuelle Herstellung einer Verbindung zwischen der Kanüle 7 und dem Hohlkörper 8 ermöglicht, wird außerdem eine sehr gute Blutschonung dadurch erreicht, dass das Blut nur mit wenigen verschiedenen Materialen in Kontakt tritt. Wie besonders gut aus Figur 3 ersichtlich ist, mündet der Hohlkörper 8, also der Pumpenauslass 6, vorzugsweise direkt in einen Pumpenraum 46 der Blutpumpe 3. In dem Pumpenraum 46 ist beispielsweise der Pumpenrotor der Blutpumpe 3 angeordnet. Der Pumpenraum 46 ist im gezeigten Beispiel ebenso wie der Pumpeneinlass 5 und der Pumpenauslass 6 durch die Außenwand des Pumpengehäuses 4 definiert. Der Kanal 16 des Pumpenauslasses 6 und der Pumpenraum 46 sind also durch dasselbe Material, beispielsweise ein biokompatibles Metall wie Edelstahl oder Titan, umgrenzt. Vom Kanal 16 des Pumpenauslasses 6 geht das Blut direkt in den Kanal 10 des Schlauchelements 9 der Kanüle 7 über, tritt bei diesem Übergang also nur mit dem Material des Pumpenauslasses 6 und dem Material des Schlauchelements 9 in Kontakt und mit keinen weiteren Materialien.

In dem in Figur 3 gezeigten Beispiel ist der Pumpenraum 46 in Form einer Spiralkammer ausgestaltet, die sich zum Pumpenauslass 6 hin verbreitert, wobei die Längsachse des Pumpenauslasses 6 senkrecht zur Drehachse des Pumpenrotors verläuft und zudem seitlich zu dieser Drehachse versetzt ist (tangentialer Auslass). Natürlich sind aber auch anderer Konfigurationen der Blutpumpe 3 möglich,

### Bezugszeichenliste:

- 1: Blutpumpensystem
- 2: Kanülensystem
- 3: Blutpumpe
- 4: Pumpengehäuse
- 5: Pumpeneinlass
- 6: Pumpenauslass
- 7: Kanüle
- 8: Hohlkörper
- 9: Schlauchelement
- 10: Kanal
- 11: vorderer Endbereich
- 12: Aufnahmebereich
- 13: vorderstes Ende
- 14: Eingangsöffnung
- 15: vorderer Endbereich
- 16: Anschlag
- 17: Verbreiterung
- 18: Oberfläche
- 19: Spannring
- 20: axialer Überlappungsbereich
- 21: Oberfläche
- 22: Oberfläche
- 23: Oberfläche
- 24: Oberfläche
- 25: vorderstes Ende
- 26: Oberfläche
- 27: Zwischenraum
- 28: Dichtring
- 29: hinteres Ende
- 30: hinteres Ende
- 31: Oberfläche
- 32: Oberfläche
- 33: axialer Überlappungsbereich
- 34: Bedienelement
- 35: Grundkörper
- 36: Grifffläche
- 37: Innenbereich
- 38: Aufnahmebereich
- 39: Aufnahmebereich
- 40: Rastelement (Rastarm)
- 41: Rastzahn
- 42: Gegenrastelement
- 43: Rastfläche
- 44: Loch
- 45: Dichtring
- 46: Pumpenraum
- 47: Außenwand

## Patentansprüche

1. Kanüle (7) zum Leiten einer Flüssigkeit, insbesondere Blut, umfassend:
- ein Schlauchelement (9) mit einem vorderen Endbereich (11) und einem hinteren Endbereich, wobei ein Kanal (10) von dem vorderen Endbereich (11) des Schlauchelements (9) bis zu dem hinteren Endbereich des Schlauchelements (9) durch das Schlauchelement (9) hindurch verläuft,
- einen Spannring (19), wobei der Spannring (19) mit dem vorderen Endbereich (11) des Schlauchelements (9) axial überlappt und mit dem vorderen Endbereich (11) des Schlauchelements (9) fest verbunden ist, wobei in einem Grundzustand der Kanüle (7), in dem auf den vorderen Endbereich (11) des Schlauchelements (9) und auf den Spannring (19) keine äußeren Kräfte einwirken, das Schlauchelement (9) durch eine von dem Spannring (19) auf den vorderen Endbereich (11) des Schlauchelements (9) ausgeübte radial nach außen gerichtete Kraft vorgespannt ist, wobei die Kanüle (7) einen Dichtring (28) umfasst, wobei der Dichtring (28) mit dem vorderen Endbereich (11) des Schlauchelements (9) axial überlappt und axial hinter dem Spannring (19) angeordnet ist.

2. Kanüle (7) gemäß Anspruch 1, **dadurch gekennzeichnet, dass** das Schlauchelement (9) durch den Spannring (19) im vorderen Endbereich (11) des Schlauchelements (9) elastisch aufgeweitet ist.

3. Kanüle (7) nach einem der vorangehenden Ansprüche, **dadurch gekennzeichnet, dass** in dem Grundzustand der Kanüle (7) ein Innendurchmesser des Schlauchelements (9) innerhalb des vorderen Endbereichs (11) zum Spannring (19) hin zunimmt.

4. Kanüle (7) nach Anspruch 1, **dadurch gekennzeichnet, dass** der Dichtring (28) von dem Spannring (19) axial beabstandet ist.

5. Kanüle (7) nach einem der Ansprüche 1 oder 4, **dadurch gekennzeichnet, dass** in dem Grundzustand der Kanüle (7), in dem zudem keine äußeren Kräfte auf den Dichtring (28) einwirken, ein Innendurchmesser des Schlauchelements (9) am Spannring (19) größer als ein Innendurchmesser des Schlauchelements (9) am Dichtring (28) ist.

6. Kanüle (7) gemäß einem der Ansprüche 1 oder 5, **dadurch gekennzeichnet, dass** der Spannring (19) steifer als der Dichtring (28) ausgestaltet ist und/oder dass der Spannring (19) aus einem festeren Material gebildet ist als der Dichtring (28).

7. Kanüle (7) gemäß einem der Ansprüche 1 oder4-6, **dadurch gekennzeichnet, dass** in dem Grundzustand der Kanüle (7) sich der Dichtring (28) zum hinteren Endbereich des Schlauchelements (9) hin verjüngt, wobei eine radial innere Oberfläche (31) des Dichtrings (28) vorzugsweise eine konische Form aufweist.

8. Kanüle (7) gemäß einem der Ansprüche 1 oder 4-7, **dadurch gekennzeichnet, dass** der Dichtring (28) relativ zum Schlauchelement (9) verschiebbar und/oder verdrehbar ist.

9. Kanüle (7) gemäß einem der vorangehenden Ansprüche, **dadurch gekennzeichnet, dass** der Spannring (19) mit dem Schlauchelement (9) stoffschlüssig verbunden ist oder mit dem Schlauchelement (9) vernäht ist.

10. Kanüle (7) gemäß einem der vorangehenden Ansprüche, **dadurch gekennzeichnet, dass** das Schlauchelement (9) aus einem Graftmaterial gefertigt ist und/oder eine textile Trägerstruktur aufweist.

11. Kanüle (7) gemäß einem der vorangehenden Ansprüche, **dadurch gekennzeichnet, dass** die Kanüle (7) ein Bedienelement (34) mit einem hülsenförmigen Grundkörper (35) aufweist, wobei der hülsenförmige Grundkörper (35) einen Innenbereich (37) definiert, wobei der vordere Endbereich (11) des Schlauchelements (9), der Spannring (19) und, sofern dieser Anspruch auf einen der Ansprüche 1 oder 4-8 zurückbezogen ist, vorzugsweise auch der Dichtring (28) in dem Innenbereich (37) des hülsenförmigen Grundkörpers (35) angeordnet sind.

12. Kanüle (7) nach Anspruch 11, **dadurch gekennzeichnet, dass** das Bedienelement (34) mindestens ein Rastelement (40), beispielsweise mindestens einen Rastarm, umfasst.

13. Kanülensystem (2), umfassend eine Kanüle (7) nach einem der vorangehenden Ansprüche und einen Hohlkörper (8), insbesondere ein Rohr, wobei der Hohlkörper (8) einen vorderen Endbereich (15) aufweist, der durch eine vordere Eingangsöffnung (14) des Kanals hindurch in den Kanal (10) des Schlauchelements (9) einführbar ist, wobei in dem Grundzustand der Kanüle (7), in dem der vordere Endbereich (15) des Hohlkörpers (8) nicht in den Kanal (10) des Schlauchelements (9) eingeschoben ist, ein Durchmesser der vorderen Eingangsöffnung (14) des Kanals (10) größer als ein Außendurchmesser des vorderen Endbereichs (15) des Hohlkörpers (8) ist.

14. Blutpumpensystem (1) mit einer Blutpumpe (3) und einem Kanülensystem (2) nach Anspruch 13, **dadurch gekennzeichnet, dass** die Blutpumpe (3) ein Pumpengehäuse (4) mit einem Pumpeneinlass (5) und einem Pumpenauslass (6) umfasst, wobei der Hohlkörper (8) des Kanülensystems den Pumpenauslass (5) oder den Pumpeneinlass (6) der Blutpumpe (3) bildet.

## Claims

1. A cannula (7) for conducting a liquid, in particular blood, comprising:
- a tube element (9) including a front end region (11) and a rear end region, wherein a channel (10) extends through the tube element (9) from the front end region (11) of the tube element (9) to the rear end region the of tube element (9);
- a tensioning ring (19), wherein the tensioning ring (19) axially overlaps the front end region (11) of the tube element (9) and is firmly connected to the front end region (11) of the tube element (9), wherein in a basic state of the cannula (7), in which no external forces act upon the front end region (11) of the tube element (9) or on the tensioning ring (19), the tube element (9) is biased by a force exerted by the tensioning ring (19) radially outward on the front end region (11) of the tube element (9), wherein the cannula (7) includes a seal ring (28), wherein the seal ring (28) axially overlaps the front end region (11) of the tube element (9) and is arranged axially behind the tensioning ring (19).

2. The cannula (7) according to claim 1, **characterized in that** the tube element (9) is elastically widened by the tensioning ring (19) in the front end region (11) of the tube element (9).

3. The cannula (7) according to one of the preceding claims, **characterized in that**, in the basic state of the cannula (7), an inner diameter of the tube element (9) within the front end region (11) increases towards the tensioning ring (19).

4. The cannula (7) according to claim 1, **characterized in that** the seal ring (28) is axially spaced from the tensioning ring (19).

5. The cannula (7) according to one of the claims 1 or 4, **characterized in that**, in the basic state of the cannula (7), in which, moreover, no external forces act upon the seal ring (28), an inner diameter of the tube element (9) at the tensioning ring (19) is larger than an inner diameter of the tube element (9) at the seal ring (28).

6. The cannula (7) according to one of the claims 1 or 5, **characterized in that** the tensioning ring (19) is designed to be stiffer than the seal ring (28) and/or **in that** the tensioning ring (19) is made of a stronger material than the seal ring (28).

7. The cannula (7) according to any one of the claims 1 or 4 to 6, **characterized in that**, in a basic state of the cannula (7), the seal ring (28) tapers towards the rear end region of the tube element (9), wherein a radial inner surface (31) of the seal ring (28) preferably has a conical shape.

8. The cannula (7) according to any one of the claims 1 or 4 to 7, **characterized in that** the seal ring (28) is axially displaceable and/or rotatable relative to the tube element (9).

9. The cannula (7) according to any of the preceding claims, **characterized in that** the tensioning ring (19) is firmly bonded to the tube element (9) or is sewn to the tube element (9).

10. The cannula (7) according to any of the preceding claims, **characterized in that** the tube element (9) is made of a graft material and/or has a textile support structure.

11. The cannula (7) according to any of the preceding claims, **characterized in that** the cannula (7) has an operating element (34) with a sleeve-shaped base body (35), wherein the sleeve-shaped base body (35) defines an inner region (37), wherein the front end region (11) of the tube element (9), the tensioning ring (19), and, inasmuch as this claim refers back to one of the claims 1 or 4 to 8, preferably also the seal ring (28) are arranged in the inner region (37) of the sleeve-shaped base body (35).

12. The cannula (7) according to claim 11, **characterized in that** the operating element (34) comprises at least one detent element (40), for example at least one detent arm.

13. A cannula system (2) comprising a cannula (7) according to any one of the preceding claims and a hollow body (8), in particular a tube, wherein the hollow body (8) includes a front end region (15) that can be inserted into the channel (10) of the tube element (9) through a front inlet opening (14) of the channel, wherein, in the basic state of the cannula (7), in which the front end region (15) of the hollow body (8) is not inserted into the channel (10) of the tube element (9), a diameter of the front inlet opening (14) of the channel (10) is larger than an outer diameter of the front end region (15) of the hollow body (8).

14. A blood pump system (1) comprising a blood pump (3) and a cannula system (2) according to claim 13, **characterized in that** the blood pump (3) comprises a pump housing (4) having a pump inlet (5) and a pump outlet (6), the hollow body (8) of the cannula system forming the pump outlet (5) or the pump inlet (6) of the blood pump (3).

## Revendications

1. Canule (7) permettant de conduire un fluide, notamment du sang, comprenant :
- un élément tubulaire (9) avec une zone d'extrémité avant (11) et une zone d'extrémité arrière, un canal (10) s'écoulant à travers l'élément tubulaire (9) à partir de la zone d'extrémité avant (11) de l'élément tubulaire (9) jusqu'à la zone d'extrémité arrière de l'élément tubulaire (9),
- une bague de serrage (19), la bague de serrage (19) se superposant axialement avec la zone d'extrémité avant (11) de l'élément tubulaire (9) et étant solidement reliée avec la zone d'extrémité avant (11) de l'élément tubulaire (9), où, dans un état de base de la canule (7), dans lequel il ne s'exerce aucune force extérieure sur la zone d'extrémité avant (11) de l'élément tubulaire (9) et sur la bague de serrage (19), l'élément tubulaire (9) est précontraint par une force radialement orientée vers l'extérieur exercée par la bague de serrage (19) sur la zone d'extrémité avant (11) de l'élément tubulaire (9), où la canule (7) comprend un joint d'étanchéité (28), où le joint d'étanchéité (28) se superpose axialement à la zone d'extrémité avant (11) de l'élément tubulaire (9) et est disposé derrière la bague de serrage (19).

2. Canule (7) selon la revendication 1, **caractérisée en ce que** l'élément tubulaire (9) est élargi de manière élastique par la bague de serrage (19) dans la zone d'extrémité avant (11) de l'élément tubulaire.

3. Canule (7) selon l'une des revendications précédentes, **caractérisée en ce que**, dans l'état de base de la canule (7), un diamètre intérieur de l'élément tubulaire (9) augmente en direction de la bague de serrage (19) à l'intérieur de la zone d'extrémité avant (11).

4. Canule (7) selon la revendication 1, **caractérisée en ce que** le joint d'étanchéité (28) est espacé axialement par rapport à l'anneau de serrage (19).

5. Canule (7) selon l'une des revendications 1 ou 4, **caractérisée en ce que**, dans l'état de base de la canule (7), dans lequel, en outre, aucune force extérieure n'agit sur le joint d'étanchéité (28), un diamètre intérieur de l'élément tubulaire (9) au niveau de la bague de serrage (19) est plus grand qu'un diamètre intérieur de l'élément tubulaire (9) au niveau du joint d'étanchéité (28).

6. Canule (7) selon l'une des revendications 1 ou 5, **caractérisée en ce que** la bague de serrage (19) est conçue plus rigide que le joint d'étanchéité (28) et/ou que la bague de serrage (19) est formée à base d'un matériau plus consistant que le joint d'étanchéité (28).

7. Canule (7) selon l'une des revendications 1 ou 4 à 6, **caractérisée en ce que**, dans l'état de base de la canule (7), le joint d'étanchéité (28) s'amenuise vers la zone d'extrémité arrière de l'élément tubulaire (9), où une surface intérieure radiale(31) du joint d'étanchéité (28) présente de préférence une forme conique.

8. Canule (7) selon l'une des revendications 1 ou 4 à 7, **caractérisée en ce que** le joint d'étanchéité (28) peut être déplacé et/ou être tourné par rapport à l'élément tubulaire (9).

9. Canule (7) selon l'une des revendications précédentes, **caractérisée en ce que** la bague de serrage (19) est reliée par complémentarité des matières avec l'élément tubulaire (9) ou est cousu avec l'élément tubulaire (9).

10. Canule (7) selon l'une des revendications précédentes, **caractérisée en ce que** l'élément tubulaire (9) est fabriqué dans un matériau greffé et/ou présente une structure de support textile.

11. Canule (7) selon l'une des revendications précédentes, **caractérisée en ce que** la canule (7) présente un élément d'actionnement (34) avec un corps de base (35) en forme de gaine, où le corps de base (35) en forme de gaine définit une zone intérieure (37), où la zone d'extrémité avant (11) de l'élément tubulaire (9), la bague de serrage (19) et, dans la mesure ou cette revendication est en relation avec l'une des revendications 1 ou 4 à 8, de préférence aussi le joint d'étanchéité (28) sont également disposés dans la zone intérieure (37) du corps de base (35) en forme de gaine.

12. Canule (7) selon la revendication 11, **caractérisée en ce que** l'élément d'actionnement (34) comprend au moins un élément de verrouillage (40), par exemple, au moins un bras de verrouillage.

13. Système de canule (2), comprenant une canule (7) selon l'une des revendications précédentes et un corps creux (8), notamment un tuyau, où le corps creux (8) présente une zone d'extrémité avant (15) qui peut être insérée dans le canal (10) de l'élément tubulaire (9) à travers un orifice d'entrée avant (14) du canal, où, dans l'état de base de la canule (7), dans lequel la zone d'extrémité avant (15) du corps creux (8) n'est pas insérée dans le canal (10) de l'élément tubulaire (9), un diamètre de l'orifice d'entrée avant (14) du canal (10) est supérieur à un diamètre extérieur de la zone d'extrémité avant (15) du corps creux (8).

14. Système de pompe à sang (1) doté d'une pompe à sang (3) et d'un système de canule (2) selon la revendication 13, **caractérisé en ce que** la pompe à sang (3) comprend un boîtier de pompe (4) avec une entrée de pompe (5) et une sortie de pompe (6), où le corps creux (8) du système de canule forme la sortie de pompe (5) ou l'entrée de pompe (6) de la pompe à sang (3).
